# EUROPÄISCHE PATENTSCHRIFT

(11) **EP 2 183 582 B1**
(45) Veröffentlichungstag und Bekanntmachung des Hinweises auf die Patenterteilung: **02.10.2013**
(21) Anmeldenummer: 08785185.3
(22) Anmeldetag: 29.07.2008
(51) Int. Cl.: G01N 27/22, G01N 33/03

(54) **VORRICHTUNG ZUM MESSEN DES ZUSTANDS EINES MESSGUTS, INSBESONDERE VON ÖLEN ODER FETTEN**
APPARATUS FOR MEASURING THE STATE OF MEASURING MATERIAL, IN PARTICULAR OF OILS OR FATS
DISPOSITIF SERVANT À MESURER L'ÉTAT D'UN PRODUIT, EN PARTICULIER D'HUILES OU DE GRAISSES

(30) Priorität: 01.08.2007 DE 102007036473
(43) Veröffentlichungstag der Anmeldung: 12.05.2010
(73) Patentinhaber: Testo AG, 79853 Lenzkirch (DE)
(72) Erfinder: HALL, Jürgen, 79877 Rötenbach (DE); MUHL, Mike, 79106 Freiburg (DE)
(74) Vertreter: Patentanwälte Westphal, Mussgnug & Partner
(86) Internationale Anmeldenummer: PCT/EP2008/006238
(87) Internationale Veröffentlichungsnummer: WO 2009/015864

(56) Entgegenhaltungen:
- DE-A1- 19 755 418
- DE-A1-102004 016 958
- DE-T5-112005 000 168
- DE-U1-202005 007 144
- US-A1- 2004 255 647
- US-A1- 2006 272 415

## Beschreibung

Die Erfindung betrifft eine Vorrichtung zum Messen des Zustands eines Messguts, insbesondere von Ölen oder Fetten, gemäß dem Oberbegriff des Patentanspruchs 1.

Bei der Zubereitung von Lebensmitteln werden häufig Öle oder Fette verwendet. Unter einem Fett wird dabei insbesondere die feste Form eines Öls verstanden. Derartige Fette oder Öle werden häufig nicht nur einmalig, sondern beispielsweise in Friteusen über einen längeren Zeitraum zum aufeinanderfolgenden Garen von unterschiedlichen Lebensmitteln verwendet. Die Frittiertemperatur des Öls oder Fetts beträgt etwa 130°C bis 180°C. Bei solchen Temperaturen unterliegt das Fett oder Öl Alterungsprozessen, wie z. B. der Oxidation durch LuftSauerstoff. Auf diesem Wege entstehen zahlreiche chemische Zersetzungsprodukte, wie z. B. Aldehyde, Ketone und Polymere, welche nicht nur eine geschmackliche Verschlechterung bewirken, sondern sich auch gesundheitsschädlich auswirken können.

Daher ist es wesentlich, die entsprechenden Öle oder Fette, insbesondere Frittieröle oder Frittierfette, regelmäßig und rechtzeitig auszutauschen, wobei darauf geachtet werden sollte, dass die Öle oder Fette weder zu früh, d. h. während die Öle oder Fette noch in einwandfreiem Zustand sind, noch zu spät, d.h. nachdem die Öle oder Fette größtenteils zerstört wurden, ausgetauscht werden. Dazu werden zum Beispiel Vorrichtungen zum Messen des Zustands von Ölen oder Fetten eingesetzt, mit denen die Öle oder Fette auf ihre elektrischen Eigenschaften hin untersucht werden. Die elektrischen Eigenschaften, insbesondere die Dielektrizitätszahl der Öle oder Fette, stellen ein verlässliches Maß für den Zerstörungsgrad des Fetts oder des Öls dar.

Die DE 10 2004 016 955 A1, DE 10 2004 016 957 A1 und DE 10 2004 016 958 A1 offenbaren jeweils eine Vorrichtung zum Messen des Zustands von Ölen oder Fetten mit einem Gehäuse, einem daran befestigten hohlen Verbindungselement und einem am gegenüberliegenden Ende des Verbindungselements angebrachten Träger zur Aufnahme eines Sensors zur Messung einer elektrischen Eigenschaft des Messguts, wobei der Sensor über mindestens eine elektrische Leitung mit einer Auswerteelektronik in Verbindung steht, welche im Bereich des Gehäuses und/oder des am Gehäuse zugewandten Ende des Verbindungselements angeordnet ist. Der Träger kann mit dem Sensor in das Öl oder Fett eingetaucht werden und ist zur Bestimmung der Dielektrizitätszahl geeignet. Dabei kommt der Sensor direkt mit dem heißen Öl oder Fett in Verbindung und ist somit starken Belastungen ausgesetzt. Zum Schutz des Sensors vor Kontakt mit dem Boden oder den Wänden des Messgefäßes, in welchem das Öl oder Fett angeordnet ist, offenbaren die genannten Druckschriften ein U-förmiges Schutzmittel, welches als Umrandung des flachen Trägers ausgestaltet ist und somit den Sensor gegen Stöße schützt.

Auch die DE 200 23 601 U1, DE 101 63 760 A1 und DE 20 2005 007 144 U1 offenbaren jeweils eine Messvorrichtung zum Messen des Zustands eines Messguts bestehend aus Ölen oder Fetten, bei welchen ein Sensor an einem freien Ende eines Verbindungselements auf einem Träger angeordnet ist, welcher in das Messgut eingetaucht werden kann. Dabei kann der Sensor von einer Abschirmung oder Abdeckung umgeben sein, welche einerseits den Sensor vor Beschädigungen bei Stößen gegen die Innenwände des Messbehälters schützen soll, zusätzlich auch beispielsweise jedoch wie in der DE 101 63 760 A1 geschildert, die Funktion erfüllen kann, parasitäre Kapazitäten wenigstens teilweise abzuschirmen, so dass die Funktionsweise des Sensors nicht beeinträchtigt wird. Auch die in diesen Druckschriften offenbarten Abschirmungen schützen den Sensor lediglich gegen Stoßbelastungen, während die Abschirmungen entweder bügelartig oder, wie in der DE 101 63 760 A1 geschildert, auch räumlich den Sensor umgebend ausgebildet sind, wobei jedoch die Abschirmung derart ausgebildet ist, dass das heiße Öl oder Fett durch die Abschirmung durchtritt und direkt den Sensor umspült.

Die verwendeten Sensoren sind in der Regel als Kondensator, insbesondere als Interdigitalkondensator ausgebildet, der aus feinen ineinandergreifenden Golddrähten oder Goldbahnen, welche insbesondere aufgedruckt oder aufgedampft werden können, besteht. Eine Vorrichtung mit einem derartigen Sensor ist ganz allgemein in der DE 197 55 418 A1 beschrieben. Diese weisen in der Regel eine vergleichsweise geringe mechanische Festigkeit auf. Mechanische Beanspruchung wie sie zum Beispiel beim Reinigen des Sensors durch Abreiben mit einem Papiertuch entsteht, kann die Dicke der Goldbahnen verändern. Zudem besteht das Problem, dass sich Öl- oder Fettreste zwischen die Strukturen einnisten können, welche zu einer Erhöhung der Grundkapazität des Senors und somit zu einem falschen Messergebnis führen. Werden die Öl- oder Fettreste nicht entfernt, altern diese kontinuierlich, wodurch der Sensor zu einer kontinuierlichen Messwertveränderung neigt.

Die Aufgabe der Erfindung besteht daher darin, eine Vorrichtung zum Messen des Zustands eines Messguts, insbesondere von Ölen und Fetten, bereitzustellen, welche eine erhöhte mechanische Stabilität aufweist und welche einfacher zu reinigen ist. Insbesondere soll gewährleistet werden, dass keine Messwertverfälschungen, insbesondere durch Messgutreste, auftreten können.

Die Aufgabe der Erfindung wird gelöst durch eine Vorrichtung zum Messen des Zustands eines Messguts, insbesondere von Ölen oder Fetten, mit den Merkmalen des Patentanspruchs 1.

Vorteilhafte Ausgestaltungen und Weiterbildungen der Erfindung sind in den Unteransprüchen angegeben.

Erfindungsgemäß ist der Sensor von einer Schutzschicht abgedeckt, die den Sensor vollständig gegen einen direkten Kontakt mit dem Messgut schützt. Der Sensor kommt somit nicht mehr direkt in Berührung mit dem heißen Messgut und ist somit weniger Belastungen ausgesetzt. Dadurch erhöht sich die Lebensdauer des Sensors. Zudem verhindert die Schutzschicht, dass sich Reste des Messguts in Kavitäten des Sensors einnisten können, da diese Kavitäten von der Schutzschicht abgedeckt werden, so dass der Sensor weniger stark verschmutzt und keine Verfälschung der Messwerte durch Reste des Messguts stattfinden. Zudem lässt sich die Vorrichtung dadurch auch einfacher reinigen. Schließlich weist die Vorrichtung eine erhöhte Stabilität auf, da der Sensor, insbesondere, falls vorhanden, die Golddrähte oder die Goldbahnen des Sensors, gegen mechanische Beanspruchungen geschützt werden.

Vorzugsweise deckt die Schutzschicht den Träger einschließlich des Sensors ab, da eine derartige Schutzschicht günstiger herzustellen ist. Insbesondere ergeben sich keine Kanten zwischen Schutzschicht und Träger, an welchen Messgut haften bleiben könnte, und auch die elektrische Leitungen zwischen Sensor und Auswerteelektronik werden geschützt.

Der Sensor erfasst vorzugsweise die Dielektrizitätszahl des Messguts, da diese mit dem Alterungszustand des Messgut, insbesondere des Öls oder Fetts, korreliert ist.

Bei einer besonders bevorzugten Ausführungsform der Erfindung ist der Sensor als Kondensator, vorzugsweise als Interdigitalkondensator, ausgebildet, da mit Hilfe eines Kondensators die Dielektrizitätszahl besonders einfach gemessen werden kann. Ein Interdigitalkondensator ermögliche eine besonders zuverlässige Messung der Dielektrizitätszahl und ist zudem gegenüber Störeinflüssen unempfindlicher.

Damit die Empfindlichkeit des Sensors nicht zu stark geschmälert wird, weist die Schutzschicht vorzugsweise eine Dicke von weniger als 10 µm, vorzugsweise eine Dicke von weniger als 1 µm auf.

Um die mechanische Stabilität der Messvorrichtung zu erhöhen, weist die Schutzschicht bei einer bevorzugten Ausführungsform der Erfindung eine Härte auf, welche größer ist als die Härte von Gold, da in der Regel die Strukturen des Sensors aus Goldleiterbahnen gefertigt werden und die Schutzschicht nicht weicher als der Sensor selbst sein sollte, um die mechanische Stabilität nicht zu verschlechtern.

Vorzugsweise ist die Schutzschicht derart ausgestattet, dass sie die kapazitive Messung des Sensors nicht beeinflusst. Dazu weist vorzugsweise die Schutzschicht einen Oberflächenwiderstand von mehr als 1 MΩ, insbesondere von mehr als 10 MΩ auf, um eine geringe Leitfähigkeit der Schutzschicht zu gewährleisten. Vorzugsweise weist die Schutzschicht weiterhin eine Permittivität von weniger als 10 auf, wobei die Permittivität der Schutzschicht insbesondere vorzugsweise kleiner ist als die Permittivität des Messguts. In einer weiteren bevorzugten Ausführungsform ist die Temperaturabhängigkeit der Permittivität der Schutzschicht kleiner als die Temperaturabhängigkeit der Permittivität des Messguts, um die Funktionsweise des Sensors nicht zu beeinflussen.

In einer vorteilhaften Ausgestaltung der Erfindung weist die Schutzschicht eine gleich große oder geringere Rauhigkeit als der Träger auf, um die Rauhigkeit des Trägers nicht zu verschlechtern. Eine geringe Rauhigkeit und somit eine glatte O-berfläche verhindern abrasive Abnutzung und die Ansammlung von Messgutresten in kleinen Kavitäten. Der Träger ist vorzugsweise aus einem Isolator, insbesondere einer Keramik gefertigt, um die Sensorbahnen gegeneinander zu isolieren. Eine Keramik weist eine Rauhigkeit von etwa 0,2 µm bis 0,8 µm auf, sodass besonders bevorzugt die Schutzschicht eine Rauhigkeit von weniger als 0,8 µm, insbesondere von weniger als 0,2 µm aufweist.

Bevorzugt ist die Schutzschicht oxidationsbeständig, zumindest oxidationsbeständig bei den üblichen Einsatztemperaturen, um zu gewährleisten, dass die Schutzschicht während der Einsatzdauer keine chemischen Veränderungen erfährt, welche die Funktionsweise des Sensors beeinträchtigen könnten.

Vorzugsweise geht die Schutzschicht, zumindest bei den üblichen Einsatztemperaturen, auch keine Reaktionen mit dem Messgut selbst, mit Zersetzungsprodukten des Messguts oder mit Reinigungsmitteln, insbesondere mit alkalischen Reinigungsmitteln, ein, um zu gewährleisten, dass einerseits während der Einsatzdauer und bei den üblichen Einsatztemperaturen keine chemischen Veränderungen der Schutzschicht auftreten, welche die Funktionsweise des Sensors beeinträchtigen, und andererseits die Messvorrichtung nach dem Einsatz mit geeigneten Reinigungsmitteln gesäubert werden kann.

Bei einer besonders bevorzugten Ausführungsform der Erfindung weist die Schutzschicht eine oleophobe Oberfläche auf, welche beispielsweise durch die Verwendung eines entsprechenden Materials für die Schutzschicht oder durch die Verwendung von Oberflächenstrukturen auf der Schutzschicht erreicht wird. Dadurch reduziert sich die Haftung des Messguts an der Oberfläche, sodass die Messvorrichtung deutlich einfacher zu reinigen ist.

Vorzugsweise wird für die Schutzschicht ein Metalloxid, ein Metallnitrid, ein Metallcarbid, eine amorphe Kohlenstoffschicht oder eine Mischverbindung aus wenigstens zwei dieser Verbindungen verwendet. Bevorzugt ist die Schutzschicht aus Oxiden von Silizium, Aluminium, Titan oder Zirkon, aus Nitriden von Titan oder Silizium oder aus Carbiden von Titan oder Silizium oder aus Mischverbindungen dieser Verbindungen hergestellt. Derartige Materialien beeinflussen die Funktionsweise des Sensors nicht, verändern sich während der Einsatzdauer und bei den üblichen Einsatztemperaturen chemisch nicht und weisen zudem eine ausreichende Härte und eine ausreichend glatte Oberfläche auf, um die mechanische Stabilität und eine einfache Reinigung des Sensors zu gewährleisten.

Die Schutzschicht wird vorzugsweise in Dünn- oder Dickschichttechnologie wie beispielsweise physical vapor deposition (PVD), chemical vapor deposition (CVD), Siebdruck, Spin-Coating, Dip-Coating, Spraying oder sonstigen Verfahren aufgebracht.

Ein Ausführungsbeispiel der Erfindung wird anhand der folgenden Figuren ausführlich erläutert. Es zeigt
- Fig. 1: eine erste Ausführungsform der erfindungsgemäßen Vorrichtung in Frontansicht,
- Fig. 2: eine vergrößerte Ansicht des unteren, einzutauchenden Bereichs der Vorrichtung aus Figur 1 und
- Fig. 3: einen Längsschnitt durch den unteren Bereich der Vorrichtung gemäß Figur 2.

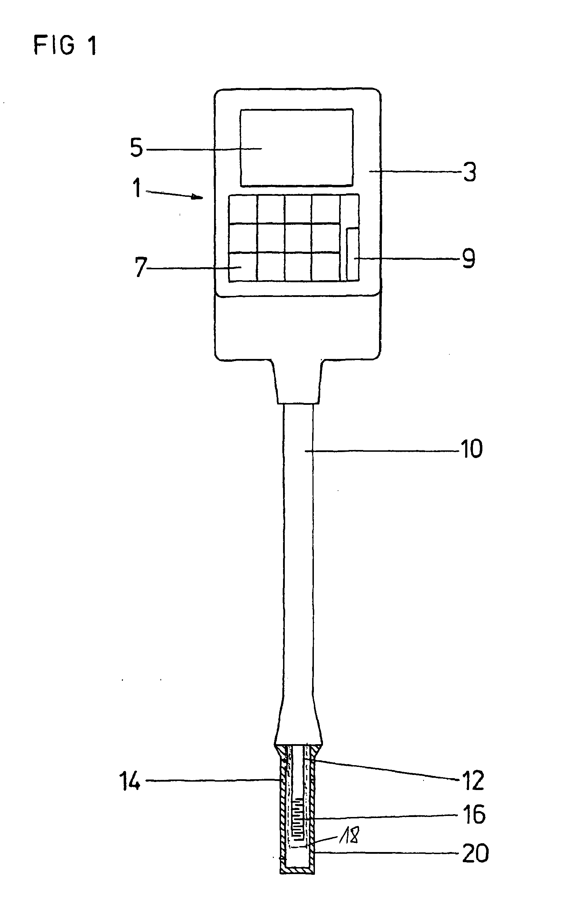

Figur 1 zeigt eine erfindungsgemäße Messvorrichtung 1 zum Messen des Zustands eines Messguts, insbesondere von Ölen oder Fetten, die in ihrem oberen Bereich ein Gehäuse 3 aufweist. Das Gehäuse weist ein Display 5 für die Anzeige von Messwerten auf. Vorzugsweise ist das Display 5 als LCD-Anzeige ausgebildet und ist zwischen graphischer Darstellung, z.B. farbliche Einstufung der Messwerte, und numerischer Darstellung umschaltbar. Zum Eingeben von Steuerungsbefehlen ist eine Tastatur 7 vorgesehen, über die Befehle an die zentrale Steuereinheit (nicht gezeigt) abgegeben werden können. Die Tastatur 7 ist vorzugsweise als Folientastatur ausgebildet. Das Gehäuse 3 kann vorzugsweise auch eine Schnittstelle 9 aufweisen, die zur Kommunikation mit externen Rechnern verwendet werden kann. Die Messvorrichtung 1 ist vorzugsweise darauf angepasst, eine Selbstkalibration durchzuführen. Während des Einsatzes der Messvorrichtung 1 dient das Gehäuse 3 gleichzeitig als Griff für die Bedienungsperson.

Von dem Gehäuse 3 ragt ein hohles Verbindungselement 10 nach unten ab, das ausreichend lang und aus einem Material mit einer schlechten Wärmeleitfähigkeit gebildet ist, so dass die empfindliche Auswerteelektronik (nicht gezeigt) der Messvorrichtung 1, die sich im Bereich des Gehäuses 3 und/oder in dem Gehäuse 3 zugewandten Bereich des Verbindungselementes 10 befindet, ausreichend vor der Hitze des zu messenden Öls bzw. Fettes geschützt ist. Durch diese Maßnahmen ist auch gewährleistet, dass die Bedienungsperson die Messungen sicher durchführen kann. Das Verbindungselement 10 ist vorzugsweise aus Edelstahl gebildet, der neben seiner geringen Wärmeleitfähigkeit auch durch seine uneingeschränkte Einsatzfähigkeit im Lebensmittelbereich geeignet ist. Das Verbindungselement 10 ist beispielsweise als rohrförmiges Bauteil ausgebildet und zur Aufnahme elektrischer Leitungen 12 geeignet, die im Inneren des Verbindungselements 10 verlaufen. Die elektrischen Leitungen 12 sind auf mindestens einem flachen Träger 14 angeordnet, der durch elektrische Isolationseigenschaften gekennzeichnet ist, beispielsweise einem Träger 14 aus Keramikmaterial.

Im unteren Bereich des Trägers 14 ist ein Sensor 16 zum Messen von elektrischen Eigenschaften des Öls bzw. Fettes angeordnet, dessen Messwerte über die elektrischen Leitungen 12 auf dem Träger 14 zur Auswerteelektronik geleitet werden. Um den unteren Bereich des Trägers 14 kann ein Schutzmittel 20 angebracht sein, das den Sensor 16 vor Kontakt mit dem Boden oder den Wänden des Messgefäßes schützt. Im vorliegenden Fall ist das Schutzmittel 20 als mit dem Verbindungselement 10 verbundene Umrandung des flachen Trägers 14 ausgestaltet und somit im wesentlichen als U-förmiger Bügel ausgebildet.

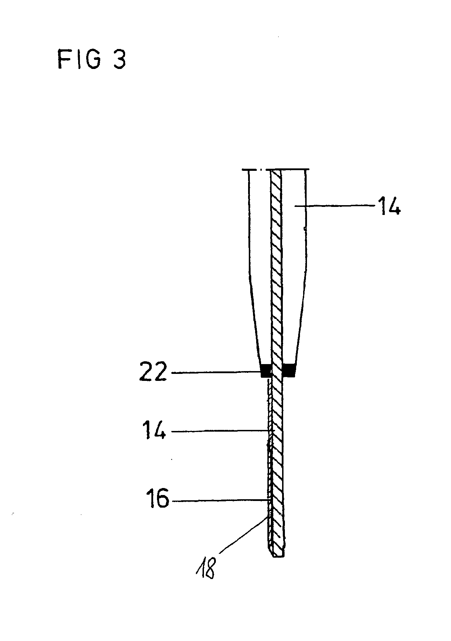

Der Zwischenbereich zwischen Träger 14 und Verbindungselement 10 ist an einer Stelle über geeignete Dichtmittel 22 isolierend abgedichtet, wie aus Figur 3 ersichtlich ist. Dort ist im unteren Endbereich des Verbindungselements 10 ein geeigneter Kleber 22, beispielsweise ein Silikonkleber, in den Zwischenbereich zwischen Träger 14 und Verbindungselement 10 eingespritzt, so dass sich diese nicht direkt berühren und somit voneinander isoliert sind. Gleichzeitig dient der Kleber 22 als Abdichtung des Verbindungselements 10, so dass kein Öl bzw. Fett in das Innere des Verbindungselements 10 eindringen kann. Die Gestaltung der Klebefläche muss sicher gegen Wassereinschlüsse sein, da ansonsten zum einen Explosionsgefahr besteht und zum anderen eine Verunreinigung des Messguts durch eingedrungene Reinigungsmittel nicht ausgeschlossen werden kann. Der Träger 14 kann als einstückiges Element bis zur Auswerteelektronik reichen, es besteht aber auch die Möglichkeit einer Entkopplung, indem mehrere Verbindungsmittel aneinandergereiht werden. Dies liefert insbesondere Vorteile hinsichtlich der Wärmebelastung der Auswerteelektronik.

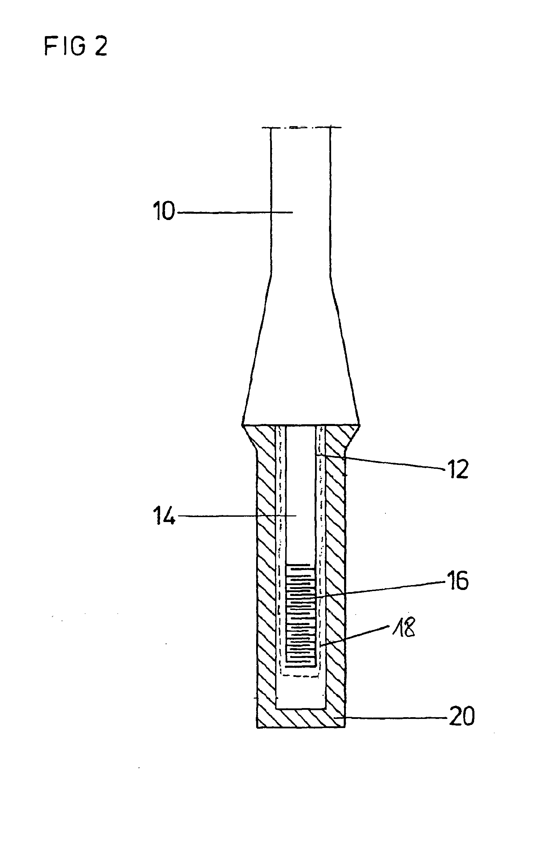

In Figur 2 ist der untere Bereich des Verbindungselements 10 und des Trägers 14 vergrößert dargestellt, der zum Eintauchen in das Messgut geeignet ist. Der Sensor 16 zur Messung der Dielektrizitätszahl besteht aus einem Kondensator, der die Dielektrizitätszahl des Öls oder Fetts misst. Vorzugsweise ist er als Interdigitalkondensator ausgebildet, der aus feinen ineinandergreifenden Golddrähten oder Goldbahnen, welche insbesondere aufgedruckt oder aufgedampft werden, besteht, die jeweils in eine der elektrischen Leitungen 12 übergehen, die zur Auswerteelektronik führen. Die Leitungen 12 bestehen aus einer feinen Auflage aus Gold bzw. Kupfer aus dem Träger 14, wobei die Auflage direkt auf das keramische Bauelement aufgedruckt ist.

Der Sensor 16 ist von einer Schutzschicht 18 abgedeckt. Die Schutzschicht 18 ist derart ausgestaltet, dass sie zumindest den Sensor 16 derart abdeckt, dass der Sensor 16 keinen direkten Kontakt zu dem Messgut, in welches die Vorrichtung 1 eingetaucht wird, hat. Wie in den Figuren dargestellt, deckt die Schutzschicht 18 vorzugsweise auch die elektrischen Leitungen 12 vollständig ab, sodass auch diese nicht in direkten Kontakt mit dem Messgut kommen. Es ist auch möglich, die Schutzschicht 18 nicht lediglich auf der Vorderseite des Trägers 14, auf welcher der Sensor 16 angeordnet ist, anzuordnen, sondern den gesamten unteren Bereich des Trägers 14 auf der Vorder- und Rückseite mit der Schutzschicht 18 abzudecken.

Die Schutzschicht 18 weist eine Dicke von weniger als 1 µm auf. Weiterhin weist die Schutzschicht eine Härte auf, welche größer ist als das Material der elektrischen Leitungen 12, vorliegend also eine Härte, welche größer ist als die Härte von Gold. Dadurch wird die mechanische Stabilität der Vorrichtung 1, insbesondere die Stabilität der Golddrähte bzw. der Goldbahnen, erhöht.

Damit die Schutzschicht 18 die kapazitive Messung mit Hilfe des Sensors 16 nicht beeinflusst, muss die Schutzschicht 18 stabile elektrische Eigenschaften aufweisen. Dazu weist die Schutzschicht 18 einen Oberflächenwiderstand von mehr als 10 MΩ auf. Zudem weist die Schutzschicht 18 eine Permittivität von weniger als 10 auf. Da die Permittivität von Ölen und Fetten etwa 3 beträgt, weist vorzugsweise die Schutzschicht 18 eine Permittivität auf, die kleiner ist als die Permitivität des Messguts und somit vorzugsweise kleiner als 3 ist. Zudem ist die Temperaturabhängigkeit der Permittivität der Schutzschicht 18 kleiner als die Temperaturabhängigkeit der Permittivität des Messguts, um die kapazitive Messung des Sensors 16 nicht zu beeinflussen.

Damit sich keine Reste des Messguts in Strukturen der Schutzschicht 18 einnisten können, weist die Schutzschicht eine Rauhigkeit von weniger als 0,2 µm auf. Die Schutzschicht ist aus Oxiden von Silizium, Aluminium, Titan oder Zirkon oder aus Nitriden von Titan oder Silizium oder aus Carbiden aus Titan oder Silizium oder aus Mischverbindungen der genannten Verbindungen hergestellt. Eine Schutzschicht 18 aus diesen Materialien ist entsprechend resistent gegen Oxidation, gegen Reaktionen mit dem Messgut oder Zersetzungsprodukten des Messguts und gegen Reinigungsmittel, insbesondere gegen alkalische, insbesondere stark alkalische Reinigungsmittel. Zudem haftet eine Schutzschicht 18 aus diesem Material gut auf der Oberfläche des Trägers 14 und des Sensors 16. Weiterhin ist die Schutzschicht 18 durch gängige Verfahren wie PVD, CVD, Siebdruck, Spin-Coating, Dip-Coating, Spraying und ähnliches, auf den Träger 14 und den Sensor aufbringbar.

### Bezugszeichenliste

- 1: Vorrichtung
- 3: Gehäuse
- 5: Display
- 7: Tastatur
- 9: Schnittstelle
- 10: Verbindungselement
- 12: elektrische Leitung
- 14: Träger
- 16: Sensor
- 18: Schutzschicht
- 20: Schutzmittel
- 22: Dichtmittel

## Patentansprüche

1. Vorrichtung (1) zum Messen des Zustands eines Messguts, insbesondere von Ölen oder Fetten, mit
einem Gehäuse (3),
einem daran befestigten hohlen Verbindungselement (10) und
einem am gegenüberliegenden Ende des Verbindungselements (10) angebrachten Träger (14) mit einem Sensor (16) zur Messung einer elektrischen Eigenschaft des Messguts,
wobei der Sensor (16) über mindestens eine elektrische Leitung (12) mit einer Auswerteelektronik in Verbindung steht, welche im Bereich des Gehäuses (3) und/oder des dem Gehäuse (3) zugewandten Endes des Verbindungselements (10) angeordnet ist, **dadurch gekennzeichnet,**
**dass** der Sensor (16) von einer Schutzschicht (18) mit einer oleophoben Oberfläche abgedeckt ist.

2. Vorrichtung nach Anspruch 1, **dadurch gekennzeichnet, dass** die Schutzschicht (18) den Träger (14) einschliesslich des Sensors (16) abdeckt.

3. Vorrichtung nach einem der vorhergehenden Ansprüche, **dadurch gekennzeichnet, dass** der Sensor (16) die Dielektrizitätszahl des Messguts erfasst.

4. Vorrichtung nach einem der vorhergehenden Ansprüche, **dadurch gekennzeichnet, dass** der Sensor (16) als Kondensator, vorzugsweise als Interdigitalkondensator, ausgebildet ist.

5. Vorrichtung nach einem der vorhergehenden Ansprüche, **dadurch gekennzeichnet, dass** die Schutzschicht (18) eine Dicke von weniger als 10 µm, vorzugsweise eine Dicke von weniger als 1 µm, aufweist.

6. Vorrichtung nach einem der vorhergehenden Ansprüche, **dadurch gekennzeichnet, dass** die Schutzschicht (18) eine Härte aufweist, welche grösser ist als die Härte von Gold.

7. Vorrichtung nach einem der vorhergehenden Ansprüche, **dadurch gekennzeichnet, dass** die Schutzschicht (18) einen Oberflächenwiderstand von mehr als 1 MΩ, vorzugsweise von mehr als 10 MΩ aufweist.

8. Vorrichtung nach einem der vorhergehenden Ansprüche, **dadurch gekennzeichnet, dass** die Schutzschicht (18) eine Permittivität von weniger als 10 aufweist, wobei die Permittivität vorzugsweise kleiner ist als die Permittivität des Messguts.

9. Vorrichttung nach einem der vorhergehenden Ansprüche, **dadurch gekennzeichnet, dass** die Temperaturabhängigkeit der Permittivität der Schutzschicht (18) kleiner ist als die Temperaturabhängigkeit der Permittivität des Messguts.

10. Vorrichtung nach einem der vorhergehenden Ansprüche, **dadurch gekennzeichnet, dass** die Schutzschicht (18) eine gleich grosse oder geringere Rauhigkeit als der Träger (14) aufweist, wobei vorzugsweise die Rauhigkeit der Schutzschicht geringer als 0,8 µm, besonders bevorzugt geringer als 0,2 µm ist.

11. Vorrichtung nach einem der vorhergehenden Ansprüche, **dadurch gekennzeichnet, dass** die Schutzschicht (18) oxidationsbeständig ist.

12. Vorrichtung nach einem der vorhergehenden Ansprüche, **dadurch gekennzeichnet, dass** die Schutzschicht (18) keine Reaktion mit dem Messgut oder Zersetzungsprodukten des Messguts oder Reinigungsmitteln eingeht.

13. Vorrichtung nach einem der vorhergehenden Ansprüche, **dadurch gekennzeichnet, dass** die Schutzschicht (18) ein Metalloxid, ein Metallnitrid, ein Metallcarbid, eine amorphe Kohlenstoffschicht oder eine Mischverbindung aus wenigstens zwei der genannten Verbindungen ist.

14. Vorrichtung nach einem der vorhergehenden Ansprüche, **dadurch gekennzeichnet, dass** die Schutzschicht (18) aus Oxiden von Silizium, Aluminium, Titan oder Zirkon oder aus Nitriden aus Titan oder Silizium oder aus Carbiden aus Titan oder Silizium oder aus Mischverbindungen aus wenigstens zwei der genannten Verbindungen hergestellt ist.

15. Vorrichtung nach einem der vorhergehenden Ansprüche, **dadurch gekennzeichnet, dass** die Schutzschicht (18) in Dünn- oder Dickschichttechnologie aufgebracht ist.

## Claims

1. Apparatus (1) for measuring the state of a measuring material, in particular oils or fats, having a housing (3),
a hollow connection element (10) fixed thereto and
a support (14) which is attached to the opposite end of the connection element (10) and has a sensor (16) for measuring an electrical property of the measuring material,
wherein the sensor (16) is connected, via at least one electrical lead (12), to an electronic evaluation system which is arranged in the region of the housing (3) and/or of the end of the connection element (10) facing the housing (3), **characterised in that** the sensor (16) is covered by a protective layer (18) having an oleophobic surface.

2. Apparatus according to claim 1, **characterised in that** the protective layer (18) covers the support (14) including the sensor (16).

3. Apparatus according to one of the preceding claims, **characterised in that** the sensor (16) records the dielectric number.

4. Apparatus according to one of the preceding claims, **characterised in that** the sensor (16) is constructed as a capacitor, preferably as an interdigital capacitor.

5. Apparatus according to one of the preceding claims, **characterised in that** the protective layer (18) has a thickness of less than 10 µm, preferably a thickness of less than 1 µm.

6. Apparatus according to one of the preceding claims, **characterised in that** the protective layer (18) has a hardness which is greater than the hardness of gold.

7. Apparatus according to one of the preceding claims, **characterised in that** the protective layer (18) has a surface resistance of more than 1 MΩ, preferably of more than 10 MΩ.

8. Apparatus according to one of the preceding claims, **characterised in that** the protective layer (18) has a permittivity of less than 10, the permittivity preferably being less than the permittivity of the measuring material.

9. Apparatus according to one of the preceding claims, **characterised in that** the temperature dependency of the permittivity of the protective layer (18) is lower than the temperature dependency of the permittivity of the measuring material.

10. Apparatus according to one of the preceding claims, **characterised in that** the protective layer (18) has a roughness which is equal to or lower than that of the support (14), the roughness of the protective layer preferably being lower than 0.8 µm, particularly preferably lower than 0.2 µm.

11. Apparatus according to one of the preceding claims, **characterised in that** the protective layer (18) is resistant to oxidation.

12. Apparatus according to one of the preceding claims, **characterised in that** the protective layer (18) does not undergo any reaction with the measuring material or decomposition products of the measuring material or cleaning compositions.

13. Apparatus according to one of the preceding claims, **characterised in that** the protective layer (18) is a metal oxide, a metal nitride, a metal carbide, an amorphous carbon layer or a mixed compound of at least two of the compounds mentioned.

14. Apparatus according to one of the preceding claims, **characterised in that** the protective layer (18) is produced from oxides of silicon, aluminium, titanium or zirconium or from nitrides of titanium or silicon or from carbides of titanium or silicon or from mixed compounds of at least two of the compounds mentioned.

15. Apparatus according to one of the preceding claims, **characterized in that** the protective layer (18) is applied in thin layer or thick layer technology.

## Revendications

1. Dispositif (1) permettant de mesurer l'état d'un produit, en particulier d'huiles ou de graisses comportant :
un boitier (3),
un élément de liaison creux (10) fixé à ce boitier et,
un support (14) monté à l'extrémité opposée de l'élément de liaison (10) et comportant un capteur (16) permettant de mesurer une caractéristique électrique du produit,
ce capteur (16) étant relié par au moins une conduite électrique (12) avec une électronique d'exploitation qui est montée dans la zone du boitier (3) et/ou de l'extrémité de l'élément de liaison (10) tournée vers le boitier (3),
**caractérisé en ce que**
le capteur (16) est recouvert d'une couche de protection (18) ayant une surface oléophobe.

2. Dispositif conforme à la revendication 1,
**caractérisé en ce que**
la couche de protection (18) recouvre le support (14) y compris le capteur (16).

3. Dispositif conforme à l'une des revendications précédentes,
**caractérisé en ce que**
le capteur (16) détecte la constante diélectrique du produit à mesurer.

4. Dispositif conforme à l'une des revendications précédentes,
**caractérisé en ce que**
le capteur (16) est réalisé sous la forme d'un condensateur, de préférence d'un condensateur interdigital.

5. Dispositif conforme à l'une des revendications précédentes,
**caractérisé en ce que**
la couche de protection (18) présente une épaisseur de moins de 10 µm, de préférence une épaisseur de moins de 1 µm.

6. Dispositif conforme à l'une des revendications précédentes,
**caractérisé en ce que**
la couche de protection (18) présente une dureté supérieure à la dureté de l'or.

7. Dispositif conforme à l'une des revendications précédentes,
**caractérisé en ce que**
la couche de protection (18) présente une résistance surfacique supérieure à 1 MΩ, de préférence supérieure à 10 MΩ.

8. Dispositif conforme à l'une des revendications précédentes,
**caractérisé en ce que**
la couche de protection (18) présente une permitivité inférieure à 10, cette permitivité étant de préférence inférieure à la permitivitié du produit à mesurer.

9. Dispositif conforme à l'une des revendications précédentes,
**caractérisé en ce que**
la variabilité avec la température de la permitivité de la couche de protection (18) est inférieure à la variabilité avec la température de la permitivité du produit à mesurer.

10. Dispositif conforme à l'une des revendications précédentes,
**caractérisé en ce que**
la couche de protection (18) présente une rugosité égale ou plus faible que celle du support (14), la rugosité de la couche de protection étant de préférence inférieure à 0.8 µm et de façon particulièrement préférentielle inférieure à 0.2 µm.

11. Dispositif conforme à l'une des revendications précédentes,
**caractérisé en ce que**
la couche de protection (18) est résistante à l'oxydation.

12. Dispositif conforme à l'une des revendications précédentes,
**caractérisé en ce que**
la couche de protection (18) ne réagit pas avec le produit à mesurer ou des produits de décomposition du produit à mesurer ou encore des agents de nettoyage.

13. Dispositif conforme à l'une des revendications précédentes,
**caractérisé en ce que**
la couche de protection (18) est un oxyde métallique, un nitrure métallique, un carbure métallique, une couche de carbone amorphe ou un mélange d'au moins deux de ces composés.

14. Dispositif conforme à l'une des revendications précédentes,
**caractérisé en ce que**
la couche de protection (18) est obtenue à partir d'oxydes de silicium, d'aluminium, de titane ou de zirconium ou de nitrures de titane ou de silicium ou de carbure de titane ou de silicium ou encore de composés mixtes d'au moins deux de ces composés.

15. Dispositif conforme à l'une des revendications précédentes,
**caractérisé en ce que**
la couche de protection (18) est appliquée par la technologie des couches minces ou des couches épaisses.
